# EUROPEAN PATENT APPLICATION

(11) **EP 4 046 673 A1**
(43) Date of publication of application: **24.08.2022**
(21) Application number: 21158801.7
(22) Date of filing: 23.02.2021
(51) Int. Cl.: A61M 5/32, A61M 5/34

(54) **NEEDLE ASSEMBLY**

(71) Applicant: Terumo Europe NV, 3001 Leuven (BE)
(72) Inventor: DANIELS, Ludo, 3700 Tongeren (BE); CASTELEYN, Pieter, 3020 Herent (BE); VALKENAERS, Hans, 2221 Booischot (BE); FRIPON, Christian, 3053 Haasrode (BE)
(74) Representative: V.O.

(57) **Abstract**

Needle assembly comprising a cannula and a hub arranged for removable connection to a syringe body, and having a proximal end and a distal end, wherein the hub is provided with a channel for establishing a fluid connection between the proximal end and the distal end, wherein the channel comprises a cannula channel part in which a proximal end of the cannula is mounted and a syringe channel part arranged to removable receive a syringe body, the needle assembly further comprising a cannula received in the cannula channel part, wherein the cannula is received in the cannula channel part such that the proximal end of the cannula extends at least partly out of the cannula channel part, wherein the cannula has a gauge of at least 18.

## Description

### FIELD

The invention generally relates to medical equipment and, more specifically, to a needle assembly for use in medical injection equipment.

A medical injection equipment, such as a syringe is generally used for directly injecting a medical liquid into a patient. The syringe typically comprises a syringe body and a needle assembly removably attached to the syringe body.

### BACKGROUND

In recent years increasing the use of viscous medical liquids, highly viscous medical liquids or non-Newtonian medical liquids has been increased, in particular for aesthetic use and/or hypodermic use. Viscous medical liquids or non-Newtonian medical liquids are advantageous for use in medical applications such as aesthetics. For example, cosmetic dermal filler products, such as, hyaluronic acid, is a highly viscous liquid and often used. In a conventional needle assembly, a proximal end of the needle often rests on a needle stop, to provide for correct positioning of the needle in the hub. The needle stop is a tubular stop to which the needle end abuts, typically having an inner diameter that is smaller than the needle inner diameter.

The application of such viscous fluids with the conventional needle assemblies can be cumbersome however. Using a conventional needle assembly with such a needle stop often results in pressure peaks during use, due to the blocking effect of the needle stop, when the physician pushes the plunger of the syringe. Such pressure peaks make it difficult for the physician to push the plunger, it is difficult for the physician to predict the force with which he has to push the plunger, and keeping a constant pushing force is practically not possible. When feeling a lot of resistance, the physician pushes the plunger harder, but he may encounter less resistance later, and then feeling that he pushes too hard. It is thus very inconvenient for the physician to use such a viscous fluid in a conventional needle assembly. In particular, when the physician does similar treatments during the day, he may have a sore or painful hand, or even a hand injury by handling conventional needle assemblies with the viscous fluid. Moreover, such viscous fluids are often, or most, used in cosmetic or aesthetic treatments. In particular when the treatments are done in the face of the patient, the use of larger gauge cannula, having a smaller outer diameter, is preferred to reduce any stains or marks of the treatment in the face of the patient. However, pushing a high viscous fluid through a high gauge needle, increases the pressure peaks, and the inconvenience for the physician to use it. Too high pressure peaks are not only inconvenient for the user, but may also result in hubs popping off or in hubs breaking loose from the syringe. This is highly undesirable during the treatment.

Also, conventional needle assemblies have some amount of dead space in which viscous fluid is left behind. Since such medical fluid can be very expensive, reduction of the dead space is desirable.

Syringe bodies often comply with ISO 7886-1:2017, so it is advantageous that a needle assembly, comprising a hub and a needle inserted into it, are ISO 80369-7:2016 compliant as well. As such, the hub can receive many different syringe bodies, and can be more universally used. When both the syringe body and the needle assembly are compliant with their respective ISO-norms, it can be assured that they fit to each other.

There is therefore a need for a needle assembly having improved characteristics. In particular, there is a need for a needle assembly allowing a more convenient use of viscous fluids. More in particular, there is a need for such a needle assembly that is ISO 80369-7:2016 compliant.

### SUMMARY

According to an aspect there is provided a needle assembly comprising a cannula and a hub arranged for removable connection to a syringe body, and having a proximal end and a distal end, wherein the hub is provided with a channel for establishing a fluid connection between the proximal end and the distal end, wherein the channel comprises a cannula channel part in which a proximal end of the cannula is mounted and a syringe channel part arranged to removable receive a syringe body, the needle assembly further comprising a cannula received in the cannula channel part, wherein the cannula is received in the cannula channel part such that the proximal end of the cannula extends at least partly out of the cannula channel part, wherein the cannula has a gauge of at least 18.

By providing a needle assembly wherein a proximal end of a cannula extends at least partly out of the cannula channel part, the proximal end will be in contact with the fluid at an outer surface of the proximal end as well as at an inner surface of the proximal end. The proximal end of the cannula is free and exposed to the fluid, allowing fluid or liquid to freely enter the proximal end of the cannula. Thereby, pressure peaks can be reduced, and a more constant pressure can be applied during the pushing of the plunger of the syringe body. This is in particular important for a cannula with a relatively large gauge, such as 18 or higher, preferably 25 or higher, more preferably 27 or higher, as a small cannula typically has a limited channel diameter, which may make it difficult for viscous fluid to pass through as well. Such a large gauge cannula, having a small outer diameter, is often used in cosmetic treatments to reduce any stains or marks of in the face of a patient. Preferably, the cannula has a gauge between 27g and 34G, corresponding with an outer diameter of between 0.4 - 0.2mm. With such small outer diameter, often thin wall, extra thin wall or ultra thin wall needles are used to have a cannula bore that is, despite its small outer diameter, relatively large, in particular when high viscosity fluids are used.

Advantageously, even less pressure than for a conventional needle assembly may be applied for achieving the same flow rate due to the exposed end of the cannula extending from the cannula channel part. The exposed proximal end of the cannula may allow the pressure drop over the length of the cannula to be less, since the fluid can enter the cannular unobstructed. A physician using a syringe comprising the needle assembly may experience a more constant resistance from the fluid into the exposed proximal end of the cannula, and thus may have to apply a more constant pressure, with less pressure peaks, in order to achieve a similar flow rate of the fluid to be injected. He can therefore administer the fluid more conveniently and repeat a similar treatment more often without tiring or injuring his hand. The decrease in pressure peaks may also result in a decrease in magnitude of possible pressure peaks. The hub is therefore less likely to break off or pop off from the syringe.

Optionally, the cannula may be a thin wall cannula. By providing a thin wall cannula, viscous fluid may pass more easily through the cannula. A thin wall cannula has a same outer diameter than a regular cannula, but a larger inner diameter, due to the thinner wall. The inner diameter of the cannula is the smallest cross sectional area through which a fluid has to flow through. By increasing this smallest cross sectional area, the overall range of cross sectional areas through which a fluid has to flow is increased, which also results in a pressure drop along the cannula being lowered. Furthermore, a larger inner diameter results in smaller circumference to cross sectional area rate in the cannula, causing frictional forces between an inner cannula wall and the fluid to be less influential than when a cannula with a standard inner diameter is used. Optionally or alternatively, the cannula may be an extra-thin wall cannula, more preferably an ultra-thin wall cannula, according to ISO 9626:2016.

Optionally or alternatively, the proximal end of the cannula may extend at least partly out of the cannula channel part over a distance of between about 0.5 mm to about 2 mm. Preferably, the proximal end of the cannula may extend at least partly out of the cannula channel part over a distance of about 1 mm. The above ranges result in an optimal decrease of pressure peaks. Having the needle extending further than 2 mm does no longer significantly influence the pressure drop and results in structural difficulties, as the design of the hub would have to be adapted in order to prevent unwanted collision between the extending needle end and a syringe end being inserted into the syringe channel part.

Optionally or alternatively, the exposed end of the cannula is between about 0.5mm and about 1mm, but may extend up to 2.5 mm. Preferably, the length of the exposed end is such that, upon inserting a syringe body into the syringe channel part, no contact can occur between the proximal end of the cannula and the syringe body. Otherwise the syringe body might damage the cannula, or vice versa. This may for example occur when, by accident, a non-standardized syringe is used. While inserting this syringe into the syringe channel part, the syringe may scrape past the proximal end of the cannula, and particles of the syringe body may be scraped from the syringe body. These particles may end up being injected into a patient, which may cause severe damage to the patient. Also, the effect of the cannula having a proximal end extending out of the cannula channel part is achieved, while minimizing dead space formed around the proximal end of the cannula.

Optionally or alternatively, the cannula may have a gauge of at most 34. The cannula may have an external diameter of at most 1.2 mm for 18G and/or at least 0.2 mm for 34G. The inner diameter depending on whether a normal wall, thin wall, ultra thin wall or extra thin wall is used. Whether a wall is a normal wall, a thin wall, an ultra thin wall or an extra thin wall is defined in ISO 9626:2016.

Optionally or alternatively, the proximal end of the cannula may be a beveled end. Preferably, a bevel angle formed between a beveled surface of the beveled end and a longitudinal axis of the cannula may be between 10 degrees and 70 degrees, preferably about 45°. By providing a beveled end, the change in cross sectional area when the fluid enters the cannula is less discrete. This may further decrease the pressure drop, and may result in the pressure in the hub or the magnitude of pressure peaks to be lower at a certain flow rate when compared to a cannula having a straight end. It shall however be appreciated that the beveled end is optional, and that a significant decrease in pressure and related pressure peaks can already be achieved by the proximal end of the cannula extending out of the cannula channel part.

Optionally or alternatively, the syringe channel part may be a tapered channel, such that an inner diameter of the syringe channel part near the distal end of the hub is smaller than an inner diameter of the syringe channel part near the proximal end of the hub. A tapered channel part may receive syringe bodies of variable sizes, as the part of the syringe body inserted into the tapered channel part also tapered. As such, providing a tapered syringe channel part allows the needle assembly to be used with a broad range of available syringe bodies.

Optionally or alternatively, the syringe channel part may have a length such that, when a syringe body is removably connected to the hub of the needle assembly, a limited or minimal dead space is formed in the tapered channel between a distal end of the syringe channel part and a distal end of a syringe body. Dead space is usually unavoidable due to the hub being combinable with syringe bodies of different sizes. The greater the size of the syringe body, the greater the dead space formed in the syringe channel part, due to the syringe body not protruding to the distal end of the syringe channel part. Preferably, the length of the syringe channel part may be such that, when the smallest combinable syringe is used, the dead space in the syringe channel part is almost zero. Preferably, the hub may be compliant to ISO 80369-7:2016. As such, the hub can be used in combination with many standardized syringe bodies. Use of the hub therefore does not require a specifically designed syringe and allows a user a flexible and/or broad freedom of choice regarding to the syringe body. It also provides for a relatively wide spectrum of possible applications of the hub. Advantageously, the length of the syringe channel part is at most7.5 mm, preferably, the length of the syringe channel part is 7.5 mm, the shortest length possible as being compliant with ISO 80369-7:2016. The minimal dead space can then reached when the smallest ISO-compliant syringe body is coupled to the hub. Larger syringe bodies may not extend so deeply into the syringe channel part thus providing for more dead space. Within an ISO-compliant syringe assembly, the needle assembly according to the invention may provide for a smaller dead space than conventional needle assemblies.

Optionally or additionally, the hub may comprise threads arranged for removable threaded connection to a syringe body. A threaded connection may provide for a more firm and secure connection between the hub and the syringe body. The thread, or threads may secure the hub to a syringe body by means of friction between the threads on the hub and threads of the syringe body. An advantage of a threaded connection is that an increase in pressure in the hub may result in the threads of the hub being pressed into the threads of the syringe body which increases the friction between the threads. As such, the force required to disconnect the hub from the syringe body increases as the pressure in the hub increases. Accidental separation of the hub from the syringe body is therefore less likely to occur, increasing the functional safety of the needle assembly. Furthermore, due to the needle assembly according to the invention, less pressure peaks and/or reduced pressure during use may occur, also reducing the risk of an accidental disconnection of the hub from the syringe body.

Optionally or additionally, the hub may further comprise an indicator arranged to indicate an orientation of a distal end of the cannula. The distal end of a cannula is typically beveled in order to puncture the skin of a patient. A physician has to know how the distal end is orientated in order to correctly puncture the skin. However, at gauge 18 and above the cannula may be too small in order to visually inspect the orientation of its distal end. The physician may then rely on the indicator in order to ensure a correct orientation of the cannula when puncturing the skin of a patient.

The hub may be of a polycarbonate or polymethylmetaacrylate (PMMA), or of other amorphe materials with an E-modulus between 1800-2600 MPa. Polycarbonate, as well as PMMA, is a stiff material which allows the hub to withstand high friction forces applied through contact surfaces formed between the hub and a syringe body or through a threaded connection formed between the hub and a syringe body. PMMA might be somewhat more impact resistant than polycarbonate, but both materials have a superior performance over the conventional polypropylene material which is too weak to be usable in combination with viscous fluids to prevent breaking off or popping off of the hub from the syringe.

Optionally or preferably, the hub may be manufactured by injection-moulding. Injection-moulding is a manufacturing process which allows for cost efficient and time efficient manufacturing of the hub, even when mass-producing the hub. Optionally or preferably, the cannula may be fixedly connected to the hub with a glue cone at a distal end of the hub. The glue cone provides a secure connection between the cannula and the hub, and prevents translational or rotational movement of the cannula relative to the hub. Furthermore, applying a glue cone is a relatively simple manufacturing step. Also, by applying the glue cone at the distal end of the hub, it may be avoided that glue accidentally enters the cannula and blocks a cannula inner channel. The assembly of the hub and the cannula to form the needle assembly can therefore be done in a cost efficient and/or a time efficient matter, even when mass-producing the needle assembly.

According to an aspect there is provided a method for manufacturing a needle assembly as described hereinabove, the method comprising providing a hub having a proximal end and a distal end wherein the hub comprises a channel for establishing a fluid connection between the proximal end and the distal end, wherein the channel comprises a cannula channel part arranged to receive a cannula and a syringe channel part arranged to removable receive a syringe body, inserting an insert in the syringe channel part of the hub such that a distance between a distal surface of the insert and a proximal opening of the cannula channel part is about 0.5 mm to 2 mm, more preferably about 1 mm, inserting a cannula into the cannula channel part from the distal end of the hub until an end of the cannula contacts the insert, fixedly connecting the cannula to the hub, and removing the insert from the syringe channel part. Optionally, the cannula may be fixedly connected to the hub using a glue cone. Advantageously, the insert may be a metal insert. By providing the metal insert during the manufacturing process, the cannula can still be positioned in the cannula channel part at the required distance and/or position without the need for a cannula stop as in the prior art hubs. After manufacturing, the metal insert can be removed out of the hub via the proximal end of the syringe channel part, while the needle remains fixated at the predefined position.

According to an aspect there is provided a syringe comprising a syringe body for containing a solution and a needle assembly as described hereinabove. In use, the solution contained by the syringe body may be a dermal filler and/or the syringe may be used for dermal injections.

Further advantageous embodiments are represented in the subclaims.

### BRIEF DESCRIPTION

The disclosure is further elucidated by means of a schematic drawing. In the drawing the following figures are shown.
Fig. 1 shows a cross sectional view of a needle assembly according to the disclosure;
Fig. 2A shows a detail of the exposed end of the cannula of Fig. 1;
Figs. 2B show an alternative to the exposed end of the cannula of Fig. 2A;
Fig. 3A shows a graph of the injection force vs extension of a conventional needle assembly;
Fig. 3B shows a graph of the injection force vs extension of the needle assembly according to the disclosure;
Fig. 4 shows the needle assembly of Fig. 1 connected to a syringe body;
Fig. 5 shows a schematic perspective view of the needle assembly of Fig. 1.

### DETAILED DESCRIPTION

The figures are given by way of schematic representations of embodiments of the disclosure. Like features are denoted with the same or similar reference numbers. The figures are not necessarily drawn to scale and are to be seen as schematic.

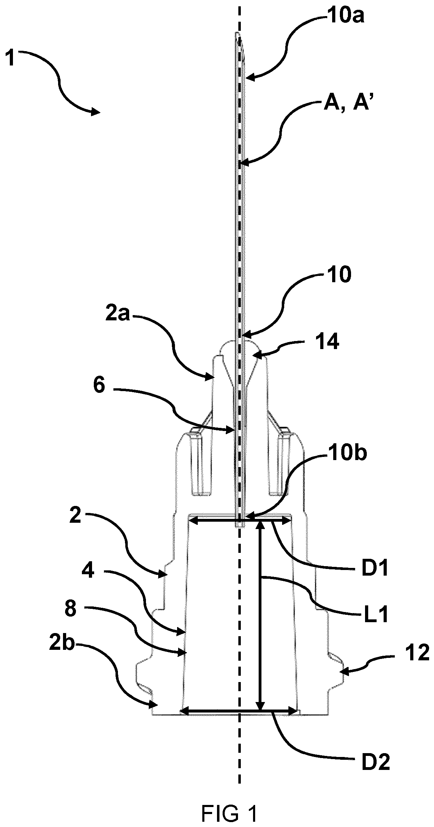

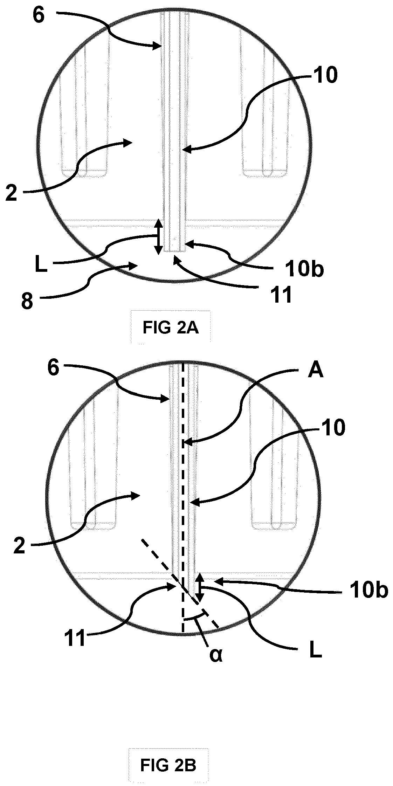

Fig. 1 shows a cross sectional view of a needle assembly 1 according to the disclosure. The needle assembly 1 comprises a hub 2 having a distal end 2a and a proximal end 2b. The hub 2 comprises a channel 4 for establishing a fluid connection between the proximal end 2b and the distal end 2a. The channel 4 comprises a cannula channel part 6 and a syringe channel part 8. A cannula 10 having a distal end 10a and a proximal end 10b is fixedly received in the cannula channel part 6. The cannula 10 has a longitudinal axis A. The hub 2 has a longitudinal axis A'. When the cannula 10 is inserted into the hub 2, the longitudinal axis A of the cannular 10 preferably coincides with the longitudinal axis A' of the hub2. The cannula 10 can be fixated into the cannula channel part 6 at the distal end 2a thereof, e.g. with a glue cone 14. The proximal end 10b of the cannula 10 extends partly out of the cannula channel part 6 and into the syringe channel part 8. Fig. 2A shows a detail of the proximal end 10b of the cannula 10 extending partly out of the cannula channel part 6 and into the syringe channel part 8, and shall be discussed more in detail below.

The cannula 10 is shown schematically and as such no specific parameters are defined regarding the cannula 10 of Fig. 1. It shall however be appreciated that the cannula 10 can be any of a cannula having a gauge of 18 or higher and the cannula 10 can be any one of a standardized wall cannula, a thin wall cannula, an extra-thin wall cannula, or an ultra-thin wall cannula.

Now referring to Fig. 2A, a detail of Fig, 1 is shown. The proximal end 10b of the cannula 10 extends partly out of the cannula channel part 6 and into the syringe channel part 8 of the hub 2. The proximal end 10b extends partly into the syringe channel part 8 over a length L. The length L can for example be between about 0.5 mm and about 1 mm. By providing that the proximal end 10b of a cannula 10 extends at least partly out of the cannula channel part 6, it is achieved that an edge of the proximal end 10b of the cannula 10 is exposed to a liquid present in the hub 2. The exposed end 10b influences the flow pattern of a liquid passing through the needle assembly 1. More specifically, the exposed end 10b decreases the pressure drop measured between the distal end 2a and the proximal end 2b of the hub 2. Therefore, less pressure has to be applied in order to achieve a certain flow rate. Further, due to the exposed end 10b of the cannula 10, it is observed that there are less pressure peaks during injection, as well as that the magnitude of eventual pressure peaks is smaller. The hub 2 is therefore less likely to break off or to pop off from a syringe body. During injection of liquid contained in the syringe body, pressure peaks can occur. The liquid contained in the syringe body is pushed out of the syringe body by a plunger contained in the syringe body. The plunger is being pushed towards a distal end of the syringe body to push the liquid out of the syringe body, into the syringe channel part 8 of the hub 2 and further into the cannula 10 such that the fluid exits the cannula 10 at its distal end 10a. In particular, when the liquid has a rather high viscosity, e.g. such as a gel-like liquid, pressure peaks may occur as it may be difficult to push the high viscosity liquid through a small diameter cannula. Due to the exposed proximal end 10b of the cannula into the syringe channel part 8, pressure peaks may be reduced.

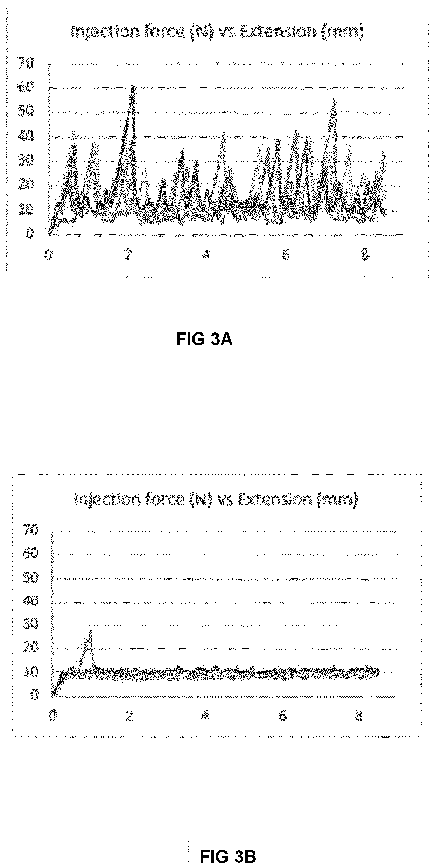

To illustrate this, in fig. 3A a force graph is shown for a conventional needle assembly, and in fig. 3B a force graph is shown for a needle assembly according to the disclosure. The injection force is the force in N to be applied to the plunger of the syringe body to push the fluid out of the syringe body through the cannula of the needle assembly. This is in fact the force that the user has to apply to the plunger. The extension is the path or length in mm that the plunger of the syringe body is displaced during the injection. For both tests, a cannula of 29G with a thin wall, according to ISO 9626:2016, is used, with a straight non-beveled end. Also, for both tests the same fluid is used, namely Hyaluronic Acid (HA). The Hyaluronic Acid dermal filler is a firm crosslinked hyaluronic acid filler and is a non-Newtonian fluid. The test fluid used comes from the same sample, such that the fluid with which the tests have been run is exactly the same. In each graph, five lines are shown, indicating that the same test has been run five times, independently of each other. The tests have been performed in accordance with ISO 7886-1:2017 "determination of force required to empty syringe". The speed of the plunger during the tests was between 10 - 15 mm/min to simulate a manual injection, i.e. by the hand of a user. As can be seen in Fig. 3A. for the conventional needle assembly, high pressure peaks are experienced, up to 55N and 60N even. Also, the average pressure is about 17N over the five tests run. In Fig. 3B, five tests have been run with the same HA fluid, the same 29G, thin walled needle, but now the cannula has an exposed proximal end that extends 1 mm into the syringe channel part. There are significantly less pressure peaks, and the maximum pressure peak observed is about 20N. Also, the average pressure over the five tests run is about 8N. Less pressure peaks and a lower average pressure significantly increases the user-friendliness of the needle assembly. This means that the user can push less hard, and that he experiences a more constant pressure. Due to the exposed end, the fluid is pushed in a more beneficial flow path towards an entrance of the cannula, thereby decreasing resistance, and thus lowering pressure and/or pressure peaks.

As shown in Fig. 2A, the length L of the protrusion of the proximal end 10b is such that the proximal end 10b extends only into the syringe channel part 8. The length L of the exposed proximal end 10b of the cannula 10, is advantageously sufficiently short such that there is no accidental contact between the exposed end 10b and a syringe body being inserted into the syringe channel part 8. Furthermore, by allowing the cannula channel part 6 to be directly in contact with the syringe channel part 8, without any additional chamber or recess in between, less additional dead space is formed and the overall dead space can be limited. In Fig. 2A described hereinabove, the proximal end 10b of the cannula 10 is a cannula end of which an end surface 11 is perpendicular to the longitudinal axis A of the cannula 10.

Fig. 2B shows a detail of an embodiment, wherein the cannula 10 extends into the syringe channel part 8 over the length L and wherein the exposed proximal end 10b of the cannula 10 is beveled. The end surface 11 is angled with respect to the longitudinal axis A of the cannula.

The beveled end 10b has a bevel angle α formed between the longitudinal axis A of the cannula 10 and a beveled surface 11 of the beveled end 10b. The bevel angle α can typically be 45°, but is advantageously between 10 degrees and 70 degrees. The beveled end 10b provides for a more continuous transition from an inner diameter of the hub to an inner diameter of the cannula 10. As such, the pressure drop measured between the distal end 2a and the proximal end 2b of the hub 2 may decrease even further, as well as less pressure peaks are being observed. The proximal end 10b being beveled therefore strengthens the effect of the proximal end 10b extending out of the cannula channel part 6.

Again with reference to Fig. 1, it is shown in this embodiment that the syringe channel part 8 is tapered. An inner diameter D1 of the syringe channel part 8 near the cannula channel part 6 towards the distal end 2a of the hub 2 is smaller than an inner diameter D2 of the syringe channel part 8 near the proximal end 2b of the hub 2. Furthermore, the syringe channel part 8 has a length L1 in longitudinal direction of the hub 2. The inner diameters D1, D2 and the length L1 are preferably compliant to ISO 80369-7:2016. When being ISO-compliant, the hub 2 is arranged to be usable with a broad range of syringe bodies. As such, the hub 2, and in particular the syringe channel part 8 is configured to be compatible with syringe bodies that are ISO 7886-1:2017 compliant. Advantageously, the length L1 of the syringe channel part is 7.5 mm. With this length, the syringe channel part 8 can receive a broad range of syringe bodies, from the smallest to the largest ISO-compliant syringe bodies. The hub 2 further comprises threads 12 at an outer side thereof arranged for a removable threaded connection to a syringe body.

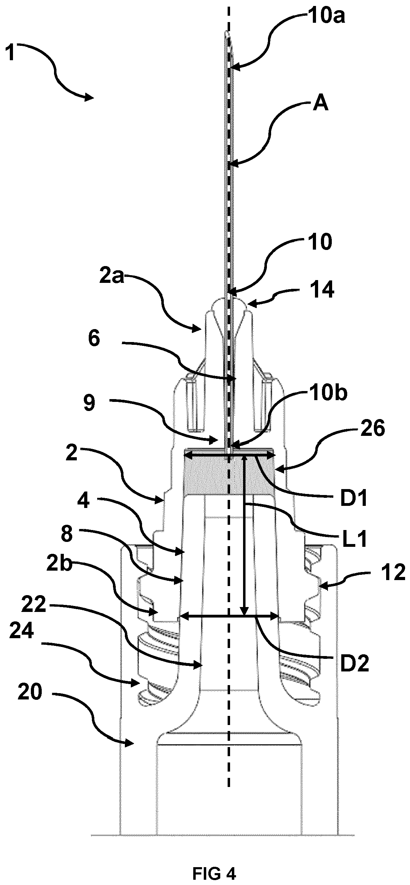

Fig. 4 shows the needle assembly 1 of Fig. 1 to which a syringe body 20 is connected. The syringe body 20 comprises a standardized male luer lock which comprises a syringe end 22 which is configured to be inserted into the syringe channel part 8 and syringe threads 24 configured to form a threaded connection with the threads 12 of the hub 2. The syringe threads 24 are provided internally to the syringe body 20 such that they can cooperate with the external threads 12 of the hub 2. The syringe channel part 8, and more specifically the inner diameters D1, D2 and the length L1, are such that a range of syringe ends of standardized syringe sizes can be received into the syringe channel part 4. Upon receiving the syringe end 22 in the syringe channel part 8, a dead space 26 is formed. Preferably, the length L1 is 7.5 mm. Some of a liquid injected using the syringe body 20 will remain in the dead space 26. It should be noted that dead space 26 is not the only dead space formed. Some of the medical solution may also remain within the syringe end 22, as well as in the cannula 10 itself. However, the dead space 26 typically forms the largest portion of the total dead space involved. Therefore, a dead space 26 that is as small as possible, is advantageous. It is thus of importance to minimize the dead space 26 such that the loss of medical solution is minimized. The length L1 of the syringe channel part 8 can therefore be chosen such that the dead space 26 is close to zero, typically 20 µl, when a smallest combinable standard ISO-syringe body is connected to the hub 2.

To inject a liquid present in the syringe body 20, pressure is applied to a plunger (not shown) of the syringe body 20. The pressure is transferred to the liquid, and causes the liquid to be ejected from the syringe body to the hub 2 and through the cannula 10. The pressure however also results in increased forces acting on the threaded connection formed between threads of the hub 2 and threads 24 of the syringe 20. If the pressure becomes too high, the threaded connection may be disrupted due to the hub deforming, popping off or braking, causing the hub to disconnect from the syringe. As described hereinabove, the proximal ending 10b of the cannula 10 extending partly out of the cannula channel part 6 causes the pressure to be reduced. Aside therefrom, the hub 2 is made from a relatively stiff material, such as PMMA or polycarbonate, compared to the commonly used polypropylene. Due to the hub 2 being made from polycarbonate, PMMA or other amorphe materials with an E-modulus between 1800-2600 MPa, the upper pressure limit the hub 2 can endure is increased. The increase of this upper pressure limit and the decrease of pressure due to the extending end of the cannula combine, and as such the risk of the hub breaking off or popping off from the syringe body is reduced greatly. A further advantage of these materials is that it allows for the hub to be manufactured using injection-moulding.

By injection moulding the hub 2, it is achieved that the needle assembly 1 can be produced in just a few steps. An example production method for the needle assembly 1 can be as follows. Step 1: injection moulding the hub 2. Step 2: Inserting a metal insert (not shown) into the syringe channel part 8. Step 3: Inserting the cannula 10 into the cannula channel part 6 until the proximal end of the cannula abuts the metal insert, and the length of the exposed end is determined. Step 4: Fixedly connect the cannula 10 to the hub 2 using a glue cone 14 at the distal end 10a of the cannula. In this production method, the metal insert serves as a stop for the cannula 10 and determines the length L of the exposed end 10b of the cannula 10. The metal insert is inserted into the syringe channel part 8 such that when the proximal end 10b of the cannula abuts the metal insert, the proximal end 10b is extending partly out of the cannula channel part. The insert is described here as a metal insert. It shall however be clear inserts of an other material may also be suitable for the production method as described hereinabove.

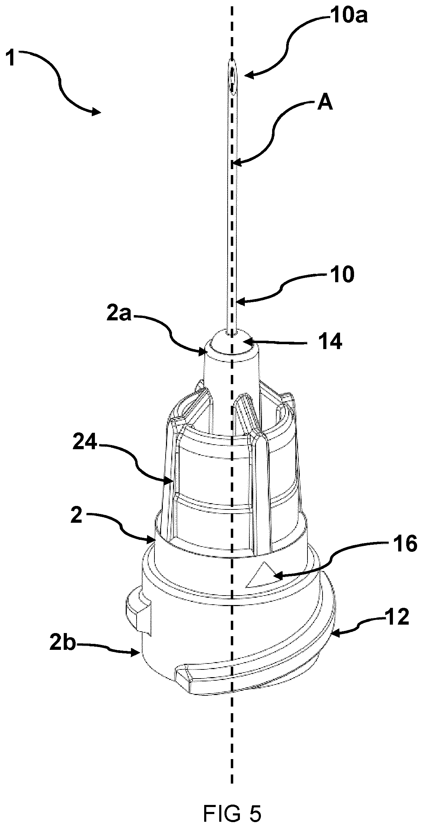

Fig. 5 shows a schematic perspective view of an outside of the needle assembly 1 of Fig. 1. The thread 12 can be seen to be spiral-shaped. Further, ribs 24 are provided to add some stiffness to the hub 2. The hub 2 further comprises an indicator 16. The indicator 16 indicates the orientation of the distal end 10a. The distal end 10a of the cannula 10 is beveled in order to puncture for example a skin of a patient. Preferably, a user such as a physician knows how the distal end 10a is orientated in order to correctly puncture the skin. However, at gauge 18 and above the cannula becomes too small in order to reliably visually inspect the orientation of the distal end 10a. The physician may then rely on the indicator 16 in order to ensure a correct orientation of the cannula 10 when puncturing the skin of a patient.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the spirit and scope of the appended claims. Further, many of the elements that are described are functional entities that may be implemented as discrete or distributed components or in conjunction with other components, in any suitable combination and location.

Many variants are possible and are comprised within the scope of the following claims.

## Claims

1. Needle assembly comprising a cannula and a hub arranged for removable connection to a syringe body, and having a proximal end and a distal end, wherein the hub is provided with a channel for establishing a fluid connection between the proximal end and the distal end, wherein the channel comprises a cannula channel part in which a proximal end of the cannula is mounted and a syringe channel part arranged to removable receive a syringe body, the needle assembly further comprising a cannula received in the cannula channel part, wherein the cannula is received in the cannula channel part such that the proximal end of the cannula extends at least partly out of the cannula channel part, wherein the cannula has a gauge of at least 18.

2. Needle assembly according to claim 1, wherein the cannula is a thin wall canula, preferably an extra-thin wall cannula, more preferably an ultra-thin wall cannula according to ISO 9626:2016.

3. Needle assembly according to claim 1 or 2, wherein the proximal end of the cannula extends at least partly out of the cannula channel part over a distance of between 0.5 mm to 2 mm.

4. Needle assembly according to claim 3, wherein the proximal end of the cannula extends at least partly out of the cannula channel part over a length of 1 mm.

5. Needle assembly according to any of the preceding claims, wherein the cannula has a gauge of at most 34, preferably has a gauge between 27 - 34, or wherein the cannula has an external diameter of at most 1.2 mm and/or at least 0.2 mm, preferably has an external diameter between 0.4 - 0.2 mm.

6. Needle assembly according to any of the preceding claims, wherein the proximal end of the cannula is a beveled end, of which an angle of the beveled surface with a longitudinal axis of the cannula is between about 10 degrees and about 70 degrees.

7. Needle assembly according to claim 6, wherein a bevel angle formed between a beveled surface of the beveled end and a longitudinal axis of the cannula is about 45°.

8. Needle assembly according to any of the preceding claims, wherein the syringe channel part is a tapered channel, such that an inner diameter of the syringe channel part near the distal end of the hub is smaller than an inner diameter of the syringe channel part near the proximal end of the hub.

9. Needle assembly according to any of the preceding claims, wherein the syringe channel part has a length such that, when a syringe body is removably connected to the hub, a dead space is formed in the tapered channel between a distal end of the syringe channel part and a distal end of a syringe body.

10. Needle assembly according to any of the preceding claims, wherein the syringe channel part has a length of at most 7.5 mm, preferably has a length of 7.5 mm.

11. Needle assembly according to any of the preceding claims, wherein the hub is compliant to ISO 80369-7:2016.

12. Needle assembly according to any of the preceding claims, wherein the hub furthermore comprises threads arranged for removable threaded connection to a syringe body.

13. Needle assembly according to any of the preceding claims, wherein the hub further comprises an indicator arranged to indicate an orientation of a distal end of the cannula.

14. Needle assembly according to any of the preceding claims, wherein the hub is of a polycarbonate.

15. Needle assembly according to any of the preceding claims, wherein the hub is manufactured by injection-moulding.

16. Needle assembly according to any of the preceding claims, wherein the cannula is fixedly connected to the hub with a glue cone.

17. Method for manufacturing a needle assembly as defined in any of the claims 1-16, the method comprising
- providing a hub having a proximal end and a distal end wherein the hub comprises a channel for establishing a fluid connection between the proximal end and the distal end, wherein the channel comprises a cannula channel part arranged to receive a cannula and a syringe channel part arranged to removable receive a syringe body;
- inserting an insert in the syringe channel part of the hub such that a distance between a distal surface of the insert and a proximal opening of the cannula channel part is about 0.5 mm to 2 mm, more preferably about 1 mm;
- inserting a cannula into the cannula channel part from the distal end of the hub until an end of the cannula contacts the insert;
- fixedly connecting the cannula to the hub; and
- removing the insert from the syringe channel part.

18. Method according to claim 17, wherein the cannula is fixedly connected to the hub using a glue cone.

19. Method according to claim 17 or 18, wherein the insert is a metal insert.

20. Syringe comprising a syringe body for containing a solution and a needle assembly as defined in any of the claims 1-16.

21. Use of the syringe as defined in claim 20 wherein the solution contained by the syringe body is a dermal filler.

22. Use of the syringe as defined in claim 20 for dermal injections.
